(19) Europäisches Patentamt
European Patent Office
Office européen des brevets

(11) Veröffentlichungsnummer: **0 109 640**
**B1**

(12) # EUROPÄISCHE PATENTSCHRIFT

(45) Veröffentlichungstag der Patentschrift: **26.02.86**

(51) Int. Cl.⁴: **C 07 D 213/68**, A 01 N 43/40

(21) Anmeldenummer: **83111357.6**

(22) Anmeldetag: **14.11.83**

(54) **Pyridinderivate, ihre Herstellung und Verwendung.**

(30) Priorität: **18.11.82 DE 3242519**

(43) Veröffentlichungstag der Anmeldung: **30.05.84 Patentblatt 84/22**

(45) Bekanntmachung des Hinweises auf die Patenterteilung: **26.02.86 Patentblatt 86/9**

(84) Benannte Vertragsstaaten: **AT BE CH DE FR GB IT LI NL SE**

(56) Entgegenhaltungen: **EP-A-0 053 306**

(73) Patentinhaber: **CELAMERCK GmbH & Co. KG, Binger Strasse 173, D-6507 Ingelheim am Rhein (DE)**

(72) Erfinder: **Schröder, Ludwig, Dr., Dipl.-Chem., Frankenstrasse 6, D-6507 Ingelheim (DE)**
Erfinder: **Mengel, Rudolf, Dr., Dipl.-Chem., Schützenpfad 22, D-6507 Ingelheim (DE)**
Erfinder: **Stransky, Werner, Dr., Dipl.-Chem., Im Hippel 24, D-6535 Gau-Algesheim (DE)**
Erfinder: **Linden, Gerbert, Dr., Dipl.-Landwirt, Turnierstrasse 44, D-6507 Ingelheim (DE)**
Erfinder: **Lust, Sigmund, Dr., Klappacher Strasse 2f, D-6100 Darmstadt (DE)**

**EP 0 109 640 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue Pyridinderivate der Formel

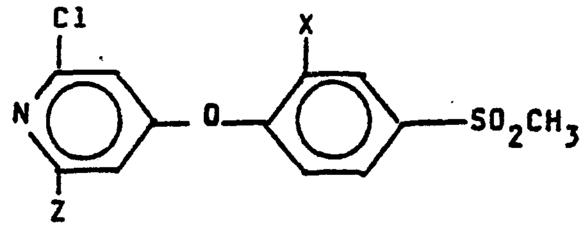

(I),

in der X für Fluor, Chlor, Brom oder $CF_3$ und Z für Chlor oder Methyl steht, ihre Herstellunq und Verwendung. Aus der europäischen Patentanmeldung Nr. 0053 306 sind Pyridinderivate der Formel

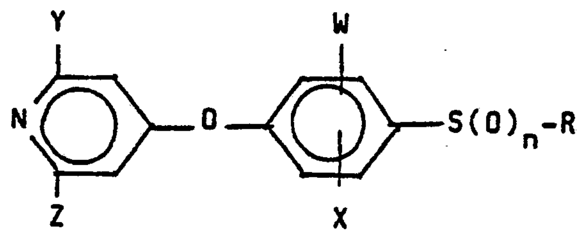

bekannt,
in der
n für 0, 1 oder 2,
R für einen gesättigten, gegebenenfalls durch ein oder mehrere Fluor- oder Chloratome substituierten, oder für einen ungesättigten aliphatischen Rest mit bis zu 4 C-Atomen, einen Benzylrest oder einen chlor- oder methylsubstituierten Benzylrest,
W und X, die gleich oder verschieden sein können, für Wasserstoff, Cl, F, Br, J oder $C_1$-$C_4$-Alkyl,
Y für Wasserstoff, Cl, F, Br oder $C_1$-$C_4$-Alkyl und
Z für Cl oder F stehen.
Diese Verbindungen haben
biozide, insbesondere herbizide Eigenschaften.

Die erfindungsgemäßen Verbindungen, die bisher noch nicht beschrieben wurden zeichnen sich überraschenderweise durch besonders günstige Wirkungsverhältnisse als Herbizide aus.

Die neuen Verbindungen können in an sich bekannter Weise hergestellt werden, indem man ein Pyridinderivat der Formel

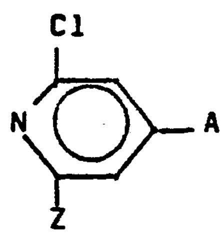

(II)

(A gleich Halogen, insbesondere Chlor, Fluor oder $NO_2$) mit einem Phenol oder Phenolat der Formel

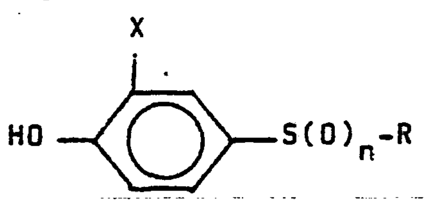

bzw.

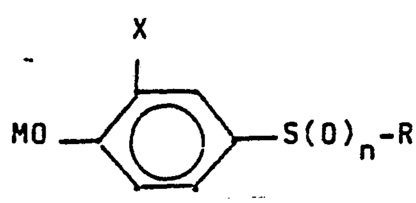

(IIIa) (IIIb).

(M gleich 1 Äquivalent eines Kations, vorzugsweise $Na^+$, $K^+$, $^1/_2\ Ca^{2+}$ oder 2, X gleich Fluor, Chlor, Brom oder $CF_3$) umsetzt und solche Reaktionsprodukte, in denen n 0 oder 1 bedeutet, zu den Verbindungen der Formel oxidiert.

Die Umsetzung der Verbindungen II und IIIa bzw. IIIb erfolgt bei Temperaturen zwischen der

Umgebungstemperatur und etwa 160° C. Man arbeitet vorzugsweise in polaren Lösungsmitteln wie Aceton, Butan-2-on, Acetonitril, Dimethylformamid, Dimethylsulfoxid bei der Siedetemperatur des Reaktionsgemischs. Bei der Umsetzung der Phenole gibt man säurebindende Mittel, z.B. Alkalicarbonate, etwa $Na_2CO_3$, $K_2CO_3$, oder Alkali- oder Erdalkalihydroxide (NaOH, KOH, $Ca(OH)_2$) zu. Um eine günstige Ausbeute zu erzielen, aenn es vorteilhaft sein, dasPhenol bzw. dessen Salze und/oder das säurebindende Mittel im Überschuß einzusetzen.

Für die Oxidation der Mercaptoverbindungen zu den Sulfonylverbindungen bzw. der Sulfinylverbindungen zu den Sulfonylverbindungen bedient man sich der für diese Reaktion üblichen Oxidationsmittel.

Im allgemeinen werden z.B. Wasserstoffperoxid, Kaliumhydrogenpersulfat oder Kaliumpermanganat verwendet.

Die Ausgangsstoffe der Formeln II, IIIa und IIIb sind bekannt oder können analog den bekannten Verbindungen erhalten werden.

Die neuen Verbindungen können außerdem nach einem neuen Verfahren erhalten werden; dieses besteht in der Umsetzung einer Verbindung der Formel

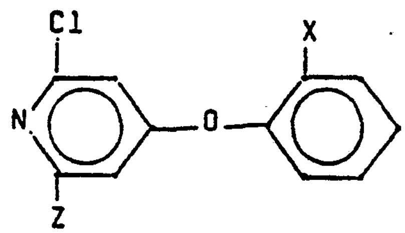

(IV)

(in der X und Z die obige Bedeutung haben) mit Chlorsulfonsäure zu den Sulfochloriden der Formel

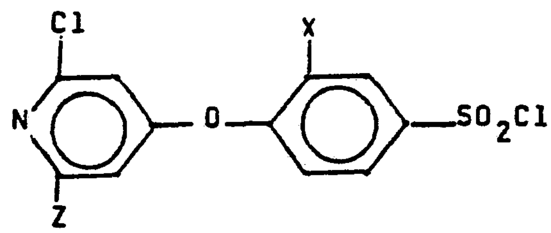

(V),

Reduktion dieser Verbindungen zur entsprechenden Sulfinsäure

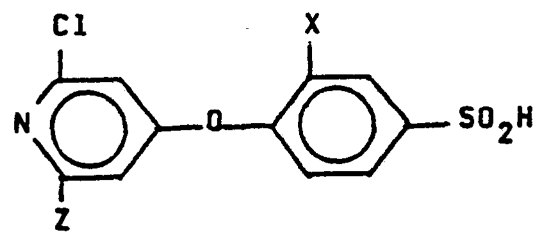

(VI),

die dann in Form eines Salzes in üblicher Weise zur Verbindung I methyliert wird.

Im einzelnen geht man so vor, daß zunächst der Pyridylether IV mit einem Überschuß von Chlorsulfonsäure bei Temperaturen oberhalb von 120°C zu V umgesetzt wird. V kann nun z.B. mit $Na_2SO_3$ bei Temperaturen zwischen 50 und 100° C, vorzugsweise zwischen 70 und 80°C, in wäßriger Suspension zu Sulfinsäuren VI reduziert werden.

Diese Säuren lassen sich in Form ihrer Salze, insbesondere ihrer Alkalisalze, oder in Gegenwart von Basen (KOH, NaOH, $Ca(OH)_2$, $Na_2CO_3$, $K_2CO_3$, $N(C_2H_5)_3$)in einem geeigneten Lösungsmittel, z.B. Wasser, Alkoholen, Dimethylformamid, Dimethylsulfoxid, Ketonen, Acetonitril oder Gemischen solcher Lösungsmittel, mit einem üblichen Methylierungsmittel zu den Verbindungen der Formel I methylieren. Als Methylierungsmittel eignen sich beispielsweise Methylhalogenide, Dimethylsulfat, p-Toluolsulfonsäuremethylester. Die Methylierung erfolgt zwischen etwa 0 und 80°C.

Die Ausgengsstoffe der Formel IV können aus Pyridinderivaten der Formel

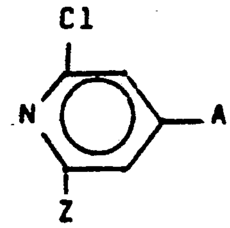

(VII)

(A Halogen, insbesondere Cl, F, oder $NO_2$) und 2-X-phenol in aprotischen Lösungsmitteln, etwa Aceton, Dimethylformemid, Acetonitril, Dimethylsulfoxid, in Gegenwert einer Base ($Na_2CO_3$, $K_2CO_3$, $Ca(OH)_2$, KOH, NaOH etc.) bei Temperaturen zwischen 0 und 160°C erhalten werden.

Die neuen Verbindungen zeichnen sich durch eine starke Wirkung gegen zahlreiche, u.a. auch gegen schwer bekampfbare Unkräuter aus.

Sie können im Vorauflauf-, aber auch im Nachauflaufverfahren in Aufwandmengen zwischen 0,01 bis 1 kg/ha eingesetzt werden, je nachdem, ob eine selektive oder vollständige Bekämpfung des Pflanzenwuchses gewünscht wird. Zur selektiven Bekämpfung von dikotylen Unkräutern in wichtigen Kulturen, wie Getreide, Mais, Baumwolle, genügen Aufwandmengen zwischen etwa 0,02 und 0,2 kg/ha.

Die neuen Verbindunqen sind erheblich wirksamer als die Verbindungen der oben genannten europäischen Anmeldung, wie der nachstehende Vergleich zeigt.

Bestimmt wurde die $ED_{90}$ d.h. 90-prozentige Schädigung der unten genanten Unkräuter im Gewächshausversuch; Vorauflaufverfahren.

Vergleichen wurden (A) 2,6-Dichlor-4-(2-chlor-4-methyl-sulfonylphenoxy)-pyridin (erfindungsgemäß, Beispiel 1) und (B) 2-Chlor-4-(2,5-dichlor-4-methylsulfonylphenoxy)-pyridin (Beispiel 3 der o.g. Europaanmeldung), als Stellungsisomere.

Die $ED_{90}$ wurde einerseits gegen 7 Dikotyle geprüft (Mittelwert), nämlich Sinapis alba, Lamium amplexicaule, Veronica hederifolia, Veronica persica, Galium aparine, Matricaria inodora, Solanum nigrum, andererseits gegen Echinocloa crus-galli.

$ED_{90}$ [kg/ha]

| Wirkstoff | 7 Dikotyle | Echinocloa crus-galli |
|---|---|---|
| A | 0,1 | 0,5 |
| B | 0,4 | 0,8 |

Es kann zweckmäßig sein, die erfindungsgemäßen Verbindungen in Kombination mit bekannten Herbiziden einzusetzen. Zu nennen sind hier z.B. Harnstoffderivate (Chlortoluron, Isoproturon, Methabenzthiazuron etc.) Triazinderivate (Atrazin, Simazin etc.), asymmetrische Triazinderivate (Metribuzin etc.), Dinitroanilinderivate (Trifluralin etc.) Acetanilidderivate (Alachlor. Metholachlor etc.), Diphenyletherderivate (Acifluorfen etc.).

Die erfindungsgemäßen Verbindungen werden in Form üblicher Zubereitungen angewendet, etwa als Spritzbrühen. Stäube, Granulate. Der Wirkstoffgehalt beträgt zwischen etwa 0,02 Gewichtsprozent (bei den anwendungsfertigen Mitteln und etwa 80 Gewichtsprozent bei den Konzentraten, die für die Anwendunq nach Bedarf verdünnt werden: insbesondere Suspensionspulvern und Emulsions- bzw. Lösungskonzentraten).

**Formulierungsbeispiele (Angaben in Gewichtsprozent):**

**a) Suspensionspulver 1**

25 % Wirkstoff gemäß Formel I
45 % Kaolin
9 % Ligninsulfonat als Dispergiermittel
1 % Natriumtetrapropylenbenzolsulfonat als Netzmittel

**b) Suspensionspulver 2**

80 % Wirkstoff gemäß Formel I
8 % Calciumligninsulfonat
5 % kolloidale Kieselsäure
5 % Natriumsulfat
2 % Natriumdiisobutylnaphthalinsulfonat

**c) Emulsionskonzentrat**

40 % Wirkstoff gemäß Formel I
25 % Shellsol A (flüssiges Gemisch aromatischer Kohlenwasserstoffe)
25 % N-Methylpyrrolidon
10 % Emulsogen I 40 (anionenaktiver Emulgator)

Die unter a) bis c) angegebenen Konzentrate werden für die Anwendung mit Wasser auf etwa 0,05 bis 5 Gewichtsprozent verdünnt.

Die Herstellung der neuen Verbindungen wird in den nachstehenden Beispielen näher erläutert.

**Beispiel 1**

**a) 2,6-Dichlor-4-(2-chlorphenoxy)-pyridin**

Eine gut gerührte Suspension von 193 g (1 mol) 2 6-Dichlor-4-nitro-pyridin, 128,6 g (1 mol) 2-Chlorphenol und 138 g (1 mol) $K_2CO_3$ in 2 L Aceton wird 3,5 Stunden unter Rückfluß gekocht. Anschließend läßt man unter weiterem Rühren innerhalb von 20 Minuten ca. 5 L Eiswasser einfließen. Der entstandene Kristallbrei wird abgesaugt, gründlich mit Wasser gewaschen und im Umluftschrank getrocknet.

Man erhält 2.6-Dichlor-4-(2-chlorphenoxy)-pyridin; Fp. 120-123°C (Rohprodukt), Ausbeute 90 - 95 %.

**b) 2,6-Dich1or-4-(2-chlor-4-chlorsulfonylphenoxy)pyridin**

In 600 ml (9 mol) Chlorsulfonsäure trägt man unter Rühren portionsweise 500 g (1,82 mol) 2,6-Dichlor-4-(2-chlorphenoxy)-pyridin ein. Dabei steigt die Innentemperatur auf ca. 65°C an. Nun erhöht man die Reaktionstemperatur auf 130°C. Die dabei einsetzende Gasentwicklung ist nach ca. 4 Stunden beendet. Man kühlt dann auf 70° - 80°C ab und gießt die viskose Lösung unter gutem Rühren in 5 L Eiswasser. Der entstehende Niederschlag wird abgesaugt und gründlich mit Wasser gewaschen. Der Filterkuchen wird nun in Methylenchlorid aufgenommen, die Lösung mit Natriumhydrogencarbonatlösung neutral gewaschen, getrocknet und eigeengt.

Man erhält ca. 510 g (75 % der Theorie)eines Produktes mit dem Fp. 140 - 146°C.

**c) 2,6-Dichlor-4-(2-chlor-4-hydroxrsulfinylphenoxy)pyridin**

Man erwärmt eine Lösung von 140 g (1,1 mol) $Na_2S0_3$ in 2 L Wasser auf 75°C und fügt portionsweise 373 g (1 mol) 2,6-Dichlor-4-(2-chlor-4-chlorsulfonylphenoxy)-pyridin und soviel $Na_2CO_3$ hinzu, daß ein pH von 8 - 9 erhalten bleibt. Das Sulfochlorid geht dabei in Lösung. Nach beendeter Zugabe wird noch ca. 2 Stunden bei gleicher Temperatur gerührt und anschließend von einem evtl. verbliebenen geringen Rückstand abfiltriert.

Man läßt das Filtrat nun abkühlen und säuert unter Rühren mit halbkonzentrierter Salzsäure an. Die ausfallenden Kristalle werden abgesaugt, mit Wasser gewaschen und in Äther aufgenommen. Man trocknet über $Na_2SO_4$, engt ein und erhält 335 g (99.% der Theorie) eines festen Produkts, Fp. 106-111°C.

**d) 2,6-Dichlor-4-(2-chlor-4-methylsulfonylphe-noxy)pyridin**

Man rührt eine Suspension von 338, 6 g (1 mol) 2,6-Dichlor-4-(2-chlor-4-hydroxysulfonylphenoxy)-pyridin, 138 g (Imol) $K_2CO_3$ und 167 g (1,1 mol) Methyljodid in 2 L DMF ca. 5 Stunden bei Raumtemperatur. Dann läßt man ca. 3 L Eiswasser ein-fließen. saugt ab und wäscht mit Wasser und kaltem Isopropanol.

Nach dem anschließenden Trocknen verbleiben 314 g (89 % der Theorie) eines festen Produkts, Fp. 190 -

195° C.

**Beispiel 2**

**a) 2-Chlor-4-(2-chlorphenoxy)-6-methylpyridin**

Eine Suspension von 162 g (1 mol) 2,4-Dichlor-6-methyl-pyridin, 128,6 g (1 mol) 2-Chlorphenol und 276 g (2 mol) $K_2CO_3$ wird in 1000 ml Dimethylformamid 7 Stunden unter Rückfluß gerührt. Nach dem Abkühlen versetzt man mit 3 L Eiswasser, saugt den entstehenden Niederschlag ab, wäscht gründlich mit Wasser und kristallisiert aus Isopropanol um.

Man erhält 168 g (66 % der Theorie) Produkt Fp. 74 - 77° C.

**b) 2-Chlor-4-(2-chlor-4-chlorsulfonylphenoxy)-6methylpyridin**

Die Verbindung wird analog Beispiel 1b aus 2-Chlor-4-(2-chlorphenoxy)-6-methylpyridin und Chlorsulfonsäure erhalten.

Die Ausbeute beträgt 84 % der Theorie; Fp. 145-147° C (aus Essigester).

**c) 2-Chlor-4-(2-chlor-4-hydroxysulfonylphenoxy)-6-methylpyridin**

Man erhält die Verbindung analog Beispiel Ic aus 2-Chlor-4-(2-chlor-4-chlorsulfonylphenoxy)-6-methyl-pyridin in einer Ausbeute von 84 % der Theorie; Fp. 107 - 110° C.

**d) 2-Chlor-4-(2-chlor-4-methylsulfonylphenoxy)-6-methylpyridin**

Man erhält die Verbindung analog Beispiel 1 d. aus 2-Chlor-4-(2-chlor-4-hydroxysulfinylphenoxy)-6-methyl-pyridin in einer Ausbeute von 82 % der Theorie;

Fp. 191 - 193° C (aus Essigester).

**Beispiel 3**

**a) 2,6-Dichlor-4-(2-fluorphenoxy)-pyridin**

Man erhält die Verbindung in Analogie zu Beispiel 1 a) aus 2,6-Dichlor-4-nitropyridin und 2-Fluorphenol. Die Ausbeute beträgt 85 % der Theorie.

Fp: 69 - 72° C.

**b) 2,6-Dichlor-4-(2-fluor-4-chlorsulfonyl-phenoxr)-pyridin**

Man erhält die Verbindung analog Beispiel 1 b) durch Reaktion der nach 3 a) erhaltenen Verbindung mit Chlorsulfonsäure.

Die Ausbeute beträgt 55 % der Theorie.

Fp: 110 - 114° C.

**c) 2,6-Dichlor-4-(2-fluor-4-hydroxysulfinyl.-phenoxy)pyridin**

Man erhält die Verbindung analog Beispiel 1 c) durch Reduktion der nach 3 b) erhaltenen Verbindung mit $Na_2SO_3$ als farbloses Öl.

Die Ausbeute beträgt 83 % der Theorie.

**d) 2-,6-Dichlor-4-(2-fluor-4-methylsulfonyl-phenoxy)pyridin**

Man erhält die Verbindung analog Beispiel 1 d) aus der nach 3 c) gewonnenen Verbindung und Methyljodid. Die Ausbeute beträgt 44 % der Theorie.
Fp. 155 - 159°C

**Beispiel 4**

**a) 2,6-Dichlor-4-(2-brom-phenoxy)-pyridin**

Man erhält die Verbindung analog Beispiel 1 a) aus 2,6-Dichlor-4-nitropyridin und 2-Bromphenol. Die Ausbeute beträgt 91 % der Theorie.
Fp: 115 - 118°C

**b) 2,6-Dichlor-4-(2-brom-4-chlorsulfonyl-phenoxy)pyridin**

Man erhält die Verbindung analog Beispiel 1 b) aus der nach 4 a) erhaltenen Verbindung und Chlorsulfonsäure. Die Ausbeute beträgt 58 % der Theorie.
Fp: 174 - 176°C

**c) 2,6-Dichlor-4-(2-brom-4-hydroxysulfinyl-phenoxy)pyridin**

Man erhält die Verbindung analog Beispiel 1 c) durch Reduktion der nach 4 b) erhaltenen Verbindung mit Na2SO3.
Die Ausbeute beträgt 85 % der Theorie.
Fp: 116 - 119°C

**d) 2,6-Dichlor-(2-brom-4-methylsulfonyl-phenoxy)-pyridin**

Man erhält die Verbindung analog Beispiel 1 d) aus der nach 4 c) erhaltenen Verbindung und Methyljodid. Die Ausbeute beträgt 41 % der Theorie.
Fp: 200 - 203°C.
Die folgenden Verbindungen können analog den Beispielen 1 bis 4 erhalten werden:
2,6-Dichlor-4-(2-trifluormethyl-4-methylsulfonylphenoxy)-pyridin
2-Chlor-4-(2-trifluormethyl-4-methylsulfonylphenoxy)-6-methylpyridin
2-Chlor-4-(2-Fluor-4-methylsulfonylphenoxy)-6-methylpyridin
2-Chlor-4-(2-Brom-4-methylsulfonylphenoxy)-6-methylpyridin

**Patentansprüche**

1. Verbindungen der Formel

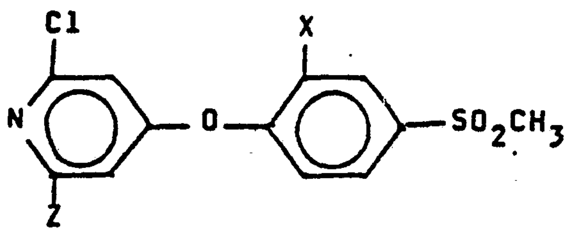 (I)

in der X für Fluor, Chlor, Brom oder $CF_3$ und Z für Chlor oder Methyl steht.
2. Biozide, insbesondere herbizide Mittel, gekennzeichnet durch einen Gehalt an mindestens einer Verbindung nach Anspruch 1.
3. Verwendung von Verbindungen nach Anspruch 1 bei der Bekämpfung unerwünschten Pflanzenwachstums.

4. Verfahren zur Herstellung von Verbindungen der Formel

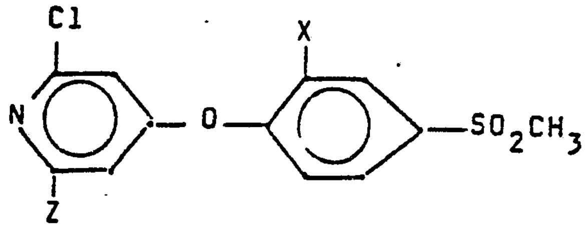

(I),

in der X für Fluor, Chlor, Brom oder $CF_3$ und Z für Chlor oder Methyl steht, dadurch gekennzeichnet, daß man a) ein Pyridinderivat der Formel

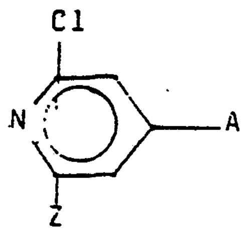

(II)

worin A Halogen oder $NO_2$ bedeutet, mit einem Phenol oder Phenolat der Formel

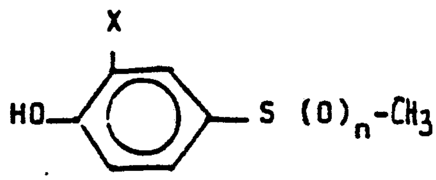

bzw.

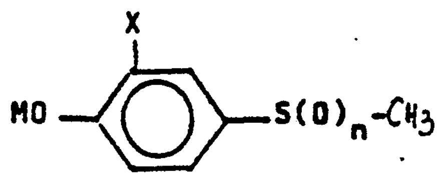

(IIIa) (IIIb)

worin M ein Äquivalent eines Kations,
X Fluor, Chlor, Brom oder $CF_3$ und n 0,1 oder 2 bedeutet, umsetzt, wobei im Falle des Phenols ein säurebindendes Mittel zugesetzt wird, und daß man solche Reaktionsprodukte, in denen n 0 oder 1 bedeutet, mit geeigneten Oxidationsmitteln zu Verbindungen der Formel I oxidiert
oder daß man
b) eine Verbindung der Formel

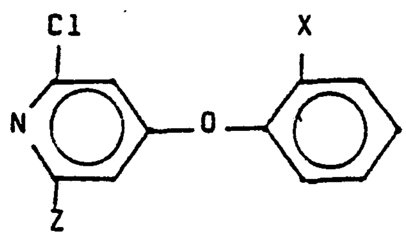

(IV)

mit Chlorsulfonsäure zu den Sulfochloriden der Formel

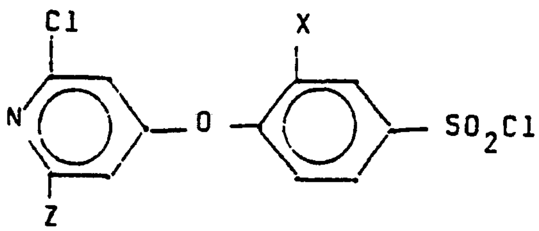

(V)

umsetzt, diese zu den entsprechenden Sulfinsäuren reduziert und die Sulfinsäuren in Form ihrer Salze methyliert.

## Claims

1. Compounds of formula

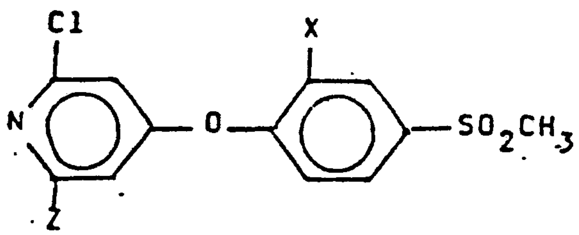

(I)

wherein X represents fluorine, chlorine, bromine or $CF_3$ and Z represents chlorine or methyl.

2. Biocidal, more particularly herbicidal agents, characterised in that they contain at least one compound as claimed in claim 1.

3. Use of compounds as claimed in claim 1 for combating undesirable plant growth.

4. Process for preparing compounds of formula

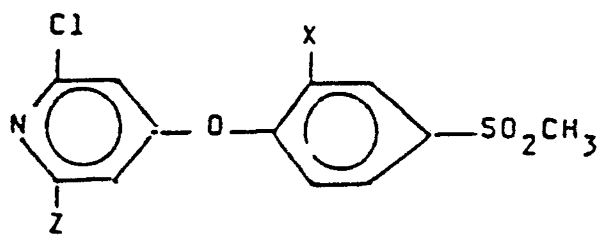

(I)

wherein X represents fluorine, chlorine, bromine or $CF_3$ and Z represents chlorine or methyl, characterised in that

a) a pyridine derivative of formula

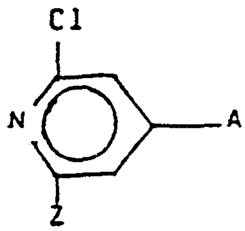

(II)

wherein A represents halogen or $NO_2$, is reacted with a phenol or phenoxide of formula

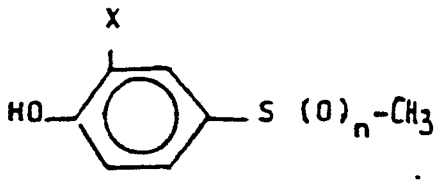

bzw.

or

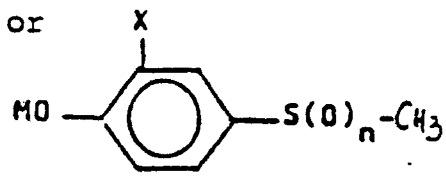

(IIIa) (IIIb)

wherein M represents an equivalent of a cation, X represents fluorine, chlorine, bromine or $CF_3$ and n represents 0, 1 or 2, whilst in the case of phenol an acid-binding agent is added, and

reaction products wherein n represents 0 or 1 are oxidised with suitable oxidising agents to form compounds of formula I

or

b) a compound of formula

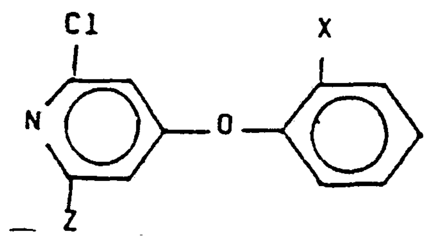

(IV)

is reacted with chlorosulphonic acid to obtain the sulphochlorides of formula

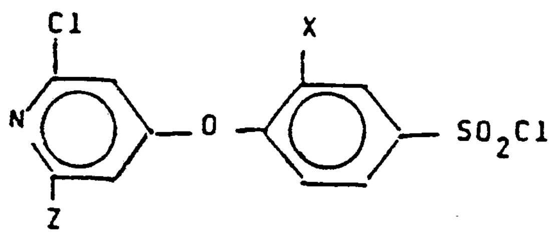

(V)

which are then reduced to form the corresponding sulphinic acids and the sulphinic acids are methylated in the form of their salts.

**Revendications**

1. Composés de formule

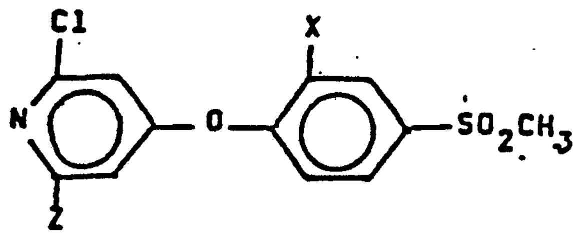 (I)

dans laquelle X remplace fluor, chlore, brome ou $CF_3$ et Z remplace chlore ou méthyle.

2. Biocide, en particulier agent herbicide, caractérisé par une teneur en au moins un composé selon la revendication 1.

3. Utilisation de composés selon la revendication 1 dans la lutte contre la croissance indésirée de plantes.

4. Procédé pour la préparation de composés de formule

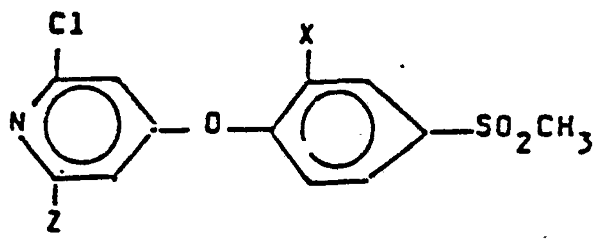 (I)

dans laquelle X remplace fluor, chlore, brome ou $CF_3$ et Z remplace chlore ou méthyle, caractérisé en ce que a) on fait réagir un dérivé de pyridine de formule

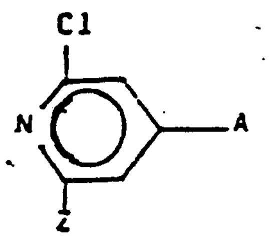 (II)

dans laquelle A signifie halogène ou $NO_2$, avec un phénol ou phénolate de formule

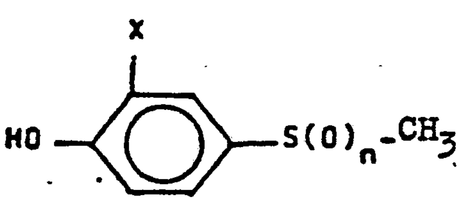 ou 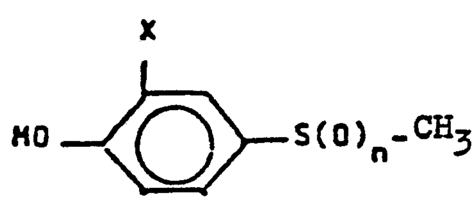

(IIIa) . (IIIb)

dans laquelle M représente un équivalent d'un cation, X représente fluor, chlore, brome ou $CF_3$ et n représente 0, 1 ou 2, dans le cas du phénol un agent fixant les acides étant ajouté, et en ce que l'on oxyde de tels produits de réaction dans lesquels n signifie 0 ou 1, au moyen d'agents d'oxydations appropriés pour donner des composés de formule I ou en ce que

b) on fait réagir un composé de formule

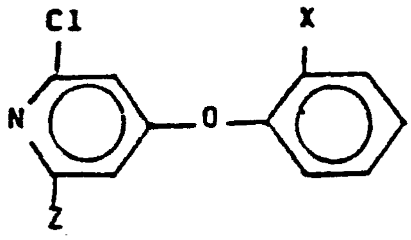

(IV)

avec l'acide chlorosulfonique pour donner les sulfochlorures de formule

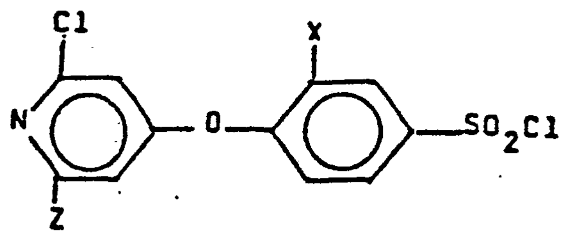

(V),

on réduit ceux-ci en les acides sulfiniques correspondants et on méthyle les acides sulfiniques sous forme de leurs sels.